(19)

**Europäisches Patentamt**
**European Patent Office**
**Office européen des brevets**

(11) **EP 4 197 429 A1**

(12) **EUROPEAN PATENT APPLICATION**

(43) Date of publication:
**21.06.2023 Bulletin 2023/25**

(21) Application number: **21214244.2**

(22) Date of filing: **14.12.2021**

(51) International Patent Classification (IPC):
*A61B 5/00* (2006.01)     *A61B 5/021* (2006.01)
*A61B 5/024* (2006.01)     *A61B 5/026* (2006.01)
*A61B 5/107* (2006.01)     *A61B 6/00* (2006.01)

(52) Cooperative Patent Classification (CPC):
**A61B 5/0044; A61B 5/02108; A61B 5/024;**
**A61B 5/026; A61B 5/107; A61B 5/72; A61B 6/504;**
A61B 5/0215; A61B 5/022

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventor: **VAN LAVIEREN, Martijn Anne**
**Eindhoven (NL)**

(74) Representative: **Philips Intellectual Property &**
**Standards**
**High Tech Campus 52**
**5656 AG Eindhoven (NL)**

(54) **SYSTEM AND METHOD FOR DETERMINING PARTIAL MASS OF AN ORGAN**

(57)     A system (100) and a method for calculating a partial mass of an organ (110) associated with a portion of a vessel (120) are provided. The system comprises one or more processors (140) configured to: receive (S110) image data comprising a temporal sequence of images (150) representing a flow of an injected contrast agent bolus through the vessel (120); receive (S120) input data representing a resting systolic blood pressure ($sBP_{rest}$) for the vessel (120); analyze (S130) the image data to determine: an average resting peak blood flow velocity ($APV_{rest}$) at the reference position (130), a diameter (D) of the vessel at the reference position (130), and a resting heart rate ($HR_{rest}$) for the vessel (120); and calculate (S140) the partial mass of the organ (110) associated with the portion of the vessel (120) based on: the determined average resting peak blood flow velocity ($APV_{rest}$), the determined diameter (D), the determined resting heart rate ($HR_{rest}$), and the resting systolic blood pressure ($sBPrest$).

FIG. 1

EP 4 197 429 A1

## Description

Technical Field

The present disclosure relates to calculating a partial mass of an organ associated with a portion of a vessel. A system, a computer-implemented method, and a computer program product, are disclosed.

Background

Coronary Artery Diseases "CAD" refers to pathologies of the coronary arteries and to the deterioration of their ability to transport oxygenated blood to the heart muscle. Ultimately, the lack of oxygen supply results in myocardial ischemia, whose symptoms can include shortness of breath, angina, or even myocardial infarction.

While obstructive CAD is well recognized as a contributing factor to the reduction of blood supply to the heart muscle, there is an increasing number of patients with evidence of ischemia and/or reports of chest pain without obstructive CAD, also referred to as Non-Obstructive Coronary Artery Disease "NOCAD".

In general, there is interest in quantifying the amount of myocardium supplied by a portion of an artery in patients with a range of pathologies relating to CAD, including obstructive CAD, and NOCAD. In patients with stable CAD, the amount of myocardium supplied by a portion of an artery distal to a stenosis in the artery is a major determinant of prognosis, as well as of the benefit of coronary revascularization. This is reported in a document by Murai, T. et al., entitled "Quantification of Myocardial Mass Subtended by a Coronary Stenosis Using Intracoronary Physiology", Circ Cardiovasc Interv. 2019; 12:e007322.

Coronary veins operate to collect, i.e. drain, oxygen-depleted blood from the myocardium. For pathologies other than CAD, there is also interest in quantifying the amount of myocardium from which blood is collected by a coronary vein. Thus, there is interest in quantifying the amount of myocardium associated with coronary vessels in general.

The amount of myocardium associated with a portion of a coronary vessel, i.e. the amount of myocardium supplied by a portion of a coronary artery distal to a reference position in the artery, or the amount of myocardium from which blood is collected by a portion of a coronary vein proximal to a reference position in the vein, is defined herein as the partial myocardial mass, or PMM. The aforementioned document by Murai, T. et al., discloses the following equation for calculating the PMM, Equation 1:

$$PMM = \frac{APV_{rest} \times D^2 \times \pi}{1.24 \times 10^{-3} \times HR_{rest} \times sBP_{rest} + 1.6}$$

In Equation 1, the parameter PMM represents the partial myocardial mass in grams. The parameter $APV_{rest}$ represents the average resting peak blood flow velocity at the reference position, in centimeters per second. The parameter D represents the diameter of the vessel at the reference position, in centimeters. The parameter $HR_{rest}$ represents the resting heart rate for the vessel, in beats per minute. The parameter $sBP_{rest}$ represents the resting systolic blood pressure for the vessel, in millimeters of mercury.

In the document by Murai, T. et al., the average resting peak blood flow velocity, $APV_{rest}$, is measured invasively using a Doppler sensor-equipped guidewire during cardiac catheterization. However, there are drawbacks to making measurements of the PMM using invasive techniques, including the need to have trained personnel available to perform the necessary cardiac catheterization.

Consequently, there is a need for improvements in making measurements of the PMM

Summary

According to the present invention, systems and corresponding methods are provided, for calculating a partial mass of an organ associated with a portion of a vessel. The portion of the vessel is defined relative to a reference position in the vessel. The system comprises one or more processors configured to:

> receive image data comprising a temporal sequence of images representing a flow of an injected contrast agent bolus through the vessel;
> receive input data representing a resting systolic blood pressure for the vessel;
> analyze the image data to determine: an average resting peak blood flow velocity at the reference position, a diameter of the vessel at the reference position, and a resting heart rate for the vessel; and
> calculate the partial mass of the organ associated with the portion of the vessel based on: the determined average resting peak blood flow velocity, the determined diameter, the determined resting heart rate, and the resting systolic blood pressure.

The organ may be for example a heart, a liver, a kidney, a brain etc., organs irrigated with blood. The vessel may be an artery or a vein. Image data may be angiographic image data provided by an X-ray imaging system or a magnetic resonance imaging system.

In an embodiment of the system, the one or more processors are configured to determine the diameter of the vessel at the reference position, by:

receiving user input specifying a location of the reference position in the image data; and
calculating the diameter of the vessel at the specified location, using the image data.

In some embodiments, the vessel comprises a stenosis, the reference position is located distal to the stenosis, and the one or more processors are configured to determine the diameter of the vessel at the reference position, by:

analysing the image data to identify a narrowing in the vessel to identify the stenosis;
specifying as the reference position, a location in the vessel distal to the stenosis; and
calculating the diameter of the vessel at the specified location, using the image data.

In some embodiments, the one or more processors are configured to determine the resting heart rate for the vessel, by:

detecting a pulsation of a feature in the image data;
extracting a heart rate signal representing the pulsation; and
determining the resting heart rate based on a period of the extracted heart rate signal.

In some embodiments, the injected contrast agent bolus is injected using an injector; and the received input data representing the resting systolic blood pressure for the vessel, comprises intra-vascular pressure data generated by the injector.

In some embodiments, the received input data representing a resting systolic blood pressure for the vessel comprises user input, or is received from a database, or is generated by:

an extra-corporeal blood pressure cuff; or
an intra-vascular pressure sensing device disposed in the vessel.

In some embodiments, the one or more processors are configured to determine the average resting peak blood flow velocity at the reference position, by:

analysing the angiographic image data, to identify a proximal position and a distal position of a front of the injected contrast agent bolus in the vessel;
estimating, from the image data, a transit length in the vessel, the transit length representing a distance travelled by the front of the injected contrast agent bolus between the proximal position and the distal position; and
calculating the average resting peak blood flow velocity at the reference position, based on the transit length and a transit time, the transit time representing the time taken for the injected contrast agent bolus to travel between the identified proximal position in the vessel, and the identified distal position in the vessel.

In some embodiments, the proximal position corresponds to a position of the front of the injected contrast agent bolus in the vessel in an earlier image in the temporal sequence, and the distal position corresponds to a position of the detected front in a later image in the temporal sequence; and the one or more processors are configured to:

determine the transit time based on the time difference between the later image and the earlier image; and
determine the transit length by:

mapping the proximal position from the earlier image to the later image to provide a mapped proximal position in the later image, or by mapping the distal position from the later image to the earlier image to provide a mapped distal position in the earlier image; and
determining as the transit length, a length of the vessel between the mapped proximal position and the distal position in the later image, or between the proximal position and the mapped distal position in the earlier image, respectively.

In some embodiments, the one or more processors are configured to calculate the partial mass for the portion of the vessel based on a correction factor, and the correction factor is determined by:

inputting into a neural network: the average resting peak blood flow velocity at the reference position, the diameter of the vessel at the reference position, the resting heart rate for the vessel, and the resting systolic blood pressure for the vessel; and
generating the correction factor in response to the inputting; and
the neural network is trained to generate the correction factor, based on the inputted average resting peak blood flow velocity, the diameter, the resting heart rate, and the resting systolic blood pressure.

In some embodiments, the neural network is trained to generate the correction factor, by:

receiving training data for a plurality of patients, the training data for each patient comprising: an average resting peak blood flow velocity at the reference position determined from image data, a diameter of the vessel at the reference position determined from image data, a resting heart rate for the vessel determined from image data, and a resting systolic blood pressure for the vessel;
receiving ground truth data corresponding to the training data, the ground truth data comprising: an average resting peak blood flow velocity at the reference position determined from an intra-vascular flow sensor, a diameter of the vessel at the reference position determined from image data, a resting heart rate for the vessel determined from a heart rate sensor, and a resting systolic blood pressure for the vessel; and
for each of a plurality of the patients:
inputting the training data into the neural network; and adjusting parameters of the neural network until a difference between i) a product of a correction factor generated by the neural network, and a partial mass calculated using the training data, and ii) a partial mass calculated using the corresponding ground truth data, meets a stopping criterion.

According to a further aspect of the present disclosure, a system for calculating a partial myocardial mass, PMM, value, is provided. The PMM value represents a mass of a myocardium associated with a portion of a coronary vessel. The portion of the coronary vessel is defined relative to a reference position in the coronary vessel. The system comprises one or more processors configured to:

- receive angiographic image data comprising a temporal sequence of images representing a flow of an injected contrast agent bolus through the coronary vessel;
- receive input data representing a resting systolic blood pressure for the coronary vessel;
- analyze the angiographic image data to determine: an average resting peak blood flow velocity at the reference position, a diameter of the coronary vessel at the reference position, and a resting heart rate for the coronary vessel; and
- calculate the PMM value for the portion of the coronary vessel, based on: the determined average resting peak blood flow velocity, the determined diameter, the determined resting heart rate, and the resting systolic blood pressure.

Since the average resting peak blood flow velocity is determined from angiographic image data, the system obviates the need to perform cardiac catheterization in order to calculate the PMM value.

In one embodiment, the one or more processors are configured to calculate the PMM value for the portion of the coronary vessel based on a correction factor, and the correction factor is determined by:

inputting into a neural network: the average resting peak blood flow velocity at the reference position, the diameter of the coronary vessel at the reference position, the resting heart rate for the coronary vessel, and the resting systolic blood pressure for the coronary vessel; and
generating the correction factor in response to the inputting; and
the neural network is trained to generate the correction factor, based on the inputted average resting peak blood flow velocity, the diameter, the resting heart rate, and the resting systolic blood pressure.

The neural network may be trained to generate the correction factor, by:

receiving training data for a plurality of patients, the training data for each patient comprising: an average resting peak blood flow velocity at the reference position determined from angiographic image data, a diameter of the coronary vessel at the reference position determined from angiographic image data, a resting heart rate for the coronary vessel determined from angiographic image data, and a resting systolic blood pressure for the coronary vessel;

receiving ground truth data corresponding to the training data, the ground truth data comprising: an average resting peak blood flow velocity at the reference position determined from an intra-vascular flow sensor, a diameter of the coronary vessel at the reference position determined from angiographic image data, a resting heart rate for the coronary vessel determined from a heart rate sensor, and a resting systolic blood pressure for the coronary vessel; and for each of a plurality of the patients:

inputting the training data into the neural network; and adjusting parameters of the neural network until a difference between i) a product of a correction factor generated by the neural network, and a PMM value calculated using the training data, and ii) a PMM value calculated using the corresponding ground truth data, meets a stopping criterion.

In some of the embodiments, the one or more processors are configured to determine the average resting peak blood flow velocity at the reference position, by:

analysing the angiographic image data, to identify a proximal position and a distal position of a front of the injected contrast agent bolus in the coronary vessel;

estimating, from the angiographic image data, a transit length in the coronary vessel, the transit length representing a distance travelled by the front of the injected contrast agent bolus between the proximal position and the distal position; and

calculating the average resting peak blood flow velocity at the reference position, based on the transit length and a transit time, the transit time representing the time taken for the injected contrast agent bolus to travel between the identified proximal position in the coronary vessel, and the identified distal position in the coronary vessel.

In some embodiments, the proximal position corresponds to a position of the front of the injected contrast agent bolus in the coronary vessel in an earlier image in the temporal sequence, and the distal position corresponds to a position of the detected front in a later image in the temporal sequence; and the one or more processors are configured to:

determine the transit time based on the time difference between the later image and the earlier image; and determine the transit length by:

mapping the proximal position from the earlier image to the later image to provide a mapped proximal position in the later image, or by mapping the distal position from the later image to the earlier image to provide a mapped distal position in the earlier image; and

determining as the transit length, a length of the coronary vessel between the mapped proximal position and the distal position in the later image, or between the proximal position and the mapped distal position in the earlier image, respectively.

In some embodiments, the one or more processors are configured to determine the diameter of the coronary vessel at the reference position, by:

receiving user input specifying a location of the reference position in the angiographic image data; and calculating the diameter of the coronary vessel at the specified location, using the angiographic image data.

In some embodiments, the coronary vessel comprises a stenosis, the reference position is located distal to the stenosis, and the one or more processors are configured to determine the diameter of the coronary vessel at the reference position, by:

analysing the angiographic image data to identify a narrowing in the coronary vessel to identify the stenosis; specifying as the reference position, a location in the coronary vessel distal to the stenosis; and calculating the diameter of the coronary vessel at the specified location, using the angiographic image data.

In some embodiments, the one or more processors are configured to determine the resting heart rate for the coronary vessel, by:

detecting a pulsation of a feature in the angiographic image data;
extracting a heart rate signal representing the pulsation; and
determining the resting heart rate based on a period of the extracted heart rate signal.

In some embodiments, the injected contrast agent bolus is injected using an injector; and the received input data representing the resting systolic blood pressure for the coronary vessel, comprises intra-vascular pressure data generated by the injector.

In some embodiments, the received input data representing a resting systolic blood pressure for the coronary vessel comprises user input, or is received from a database, or is generated by:

an extra-corporeal blood pressure cuff; or
an intra-vascular pressure sensing device disposed in the coronary vessel.

The coronary vessel may be a coronary artery; or the coronary vessel is a coronary artery comprising a stenosis, and the reference position is distal to the stenosis. The portion of the coronary vessel is distal to the reference position. The PMM value represents the mass of the myocardium supplied by the portion of the coronary artery.

The coronary vessel may be a coronary vein. The portion of the coronary vessel is proximal to the reference position, the PMM value represents the mass of the myocardium from which blood is collected by the portion of the coronary vein.

Further aspects, features, and advantages of the present disclosure will become apparent from the following description of examples, which is made with reference to the accompanying drawings.

Brief Description of the Drawings

Fig. 1 is a schematic diagram illustrating an example of a system 100 for calculating a partial myocardial mass value, in accordance with some aspects of the present disclosure.
Fig. 2 is a flowchart illustrating an example of a method for calculating a partial myocardial mass value, in accordance with some aspects of the present disclosure.
Fig. 3 is a schematic diagram illustrating a heart, including an example of a coronary vessel 120 in accordance with some aspects of the present disclosure.
Fig. 4 is a schematic diagram illustrating an example of a temporal sequence of images 150 representing a flow of an injected contrast agent bolus through a coronary vessel 120, in accordance with some aspects of the present disclosure.
Fig. 5 is a schematic diagram illustrating an earlier image and a later image in a temporal sequence of images 150, in accordance with some aspects of the present disclosure.
Fig. 6 is a schematic diagram illustrating the determination of a transit length $d_T$ in a coronary vessel 120 by mapping a proximal position Posa of a front of an injected contrast agent bolus from an earlier image in a temporal sequence of images 150 to a later image in the temporal sequence of images to provide a mapped proximal position Pos'a in the later image, in accordance with some aspects of the present disclosure.
Fig. 7 is a schematic diagram illustrating an example of a trained neural network 160 for calculating a correction factor $\phi$ for correcting a PMM value, in accordance with some aspects of the present disclosure.

Detailed Description

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a system for calculating a PMM value representing a mass of a myocardium associated with a portion of a coronary vessel. The portion of the coronary vessel is defined relative to a reference position in the coronary vessel. Reference is made to examples in which the coronary vessel is a coronary artery. In these examples, the portion of the coronary vessel is distal to the reference position, and the PMM value represents the mass of the myocardium supplied by the portion of the coronary artery. In this context, the term distal refers to positions that are downstream with respect to the reference position. However, it is also to be appreciated that the coronary vessel may alternatively be a coronary vein. In these examples, the portion of the coronary vessel is proximal to the reference position, and the PMM value represents the mass of the myocardium from which blood is collected by the portion of the coronary vein. In this context, the term proximal refers to positions that are upstream with respect to the reference position. Reference is also made to examples in which the reference position in the coronary vessel is defined by a stenosis. However, it is to be appreciated that the reference position may instead be any position in the coronary vessel. In examples in which the stenosis is in a coronary artery, the reference position may be distal to the stenosis. In other examples, the reference position may be in a coronary vein.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray™ and DVD

As mentioned above, there are drawbacks to making measurements of the PMM using invasive techniques, including the need to have trained personnel available to perform the necessary cardiac catheterization.

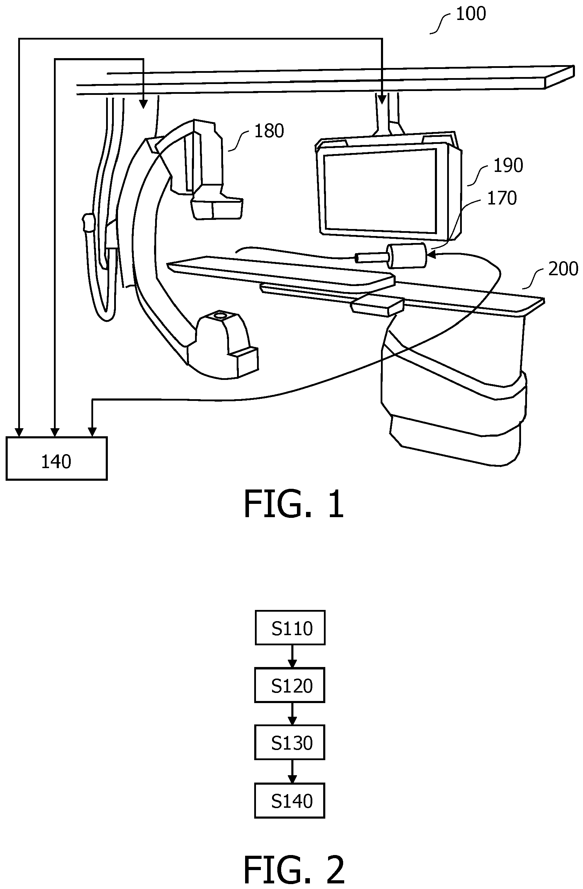

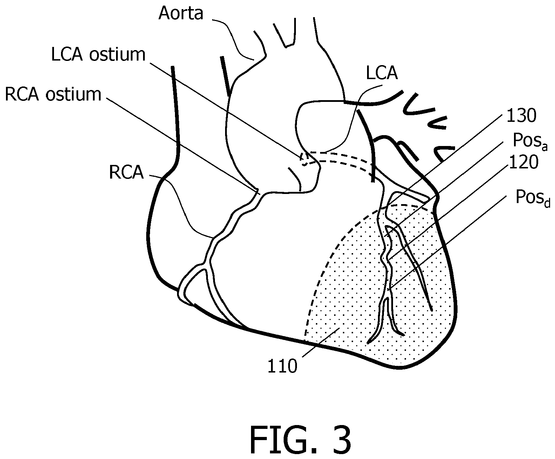

Fig. 1 is a schematic diagram illustrating an example of a system 100 for calculating a partial myocardial mass value, in accordance with some aspects of the present disclosure. Fig. 2 is a flowchart illustrating an example of a method for calculating a partial myocardial mass value, in accordance with some aspects of the present disclosure. Operations described in relation to the system 100 illustrated in Fig. 1, may also be performed in the method illustrated in Fig. 2, and vice versa. Fig. 3 is a schematic diagram illustrating a heart, including an example of a coronary vessel 120 in accordance with some aspects of the present disclosure. With reference to Fig. 1 - Fig. 3, the example system 100 is configured to calculate a partial myocardial mass, PMM, value, the PMM value representing a mass of a myocardium 110 associated with a portion of a coronary vessel 120, the portion of the coronary vessel being defined relative to a reference position 130 in the coronary vessel. The system includes one or more processors 140 configured to:

- receive S110 angiographic image data comprising a temporal sequence of images 150 representing a flow of an injected contrast agent bolus through the coronary vessel 120;
- receive S120 input data representing a resting systolic blood pressure $sBP_{rest}$ for the coronary vessel 120;
- analyze S130 the angiographic image data to determine: an average resting peak blood flow velocity $APV_{rest}$ at the reference position 130, a diameter D of the coronary vessel at the reference position 130, and a resting heart rate $HR_{rest}$ for the coronary vessel 120; and
- calculate S140 the PMM value for the portion of the coronary vessel 120, based on: the determined average resting peak blood flow velocity $APV_{rest}$, the determined diameter D, the determined resting heart rate $HR_{rest}$, and the resting systolic blood pressure $sBP_{rest}$.

Since the average resting peak blood flow velocity $APV_{rest}$ is determined from angiographic image data, the system obviates the need to perform cardiac catheterization in order to calculate the PMM value.

With reference to Fig. 3, in one example the system 100 may be used to calculate the PMM value for the portion of the coronary vessel 120 that is distal to the reference position 130. In this example, the coronary vessel is a coronary artery, and the PMM value therefore represents the mass of the myocardium 110 supplied by the portion of the coronary artery, i.e. the mass of the myocardium within the shaded region of the heart that is illustrated in Fig. 3. The portion of the coronary artery for which the PMM value is provided in this example is between the reference position 130 and the most distal extent of the coronary vasculature from the reference position 130. In this example the coronary vessel 120 is the left coronary artery LCA. The coronary vessel 120 may alternatively be another coronary artery, such as the right coronary artery RCA, for example. The reference position 130 may in general be any position in a coronary artery. In some examples, the reference position 130 is a position of a stenosis. In some examples, the reference position is distal to a stenosis. For example, the reference position may be a short distance distal to a stenosis. The reference position 120 may for example be located up to a few millimeters, or up to twenty millimeters distal to a stenosis in order to avoid the impact of turbulent flow generated by the stenosis on the measured average resting peak blood flow velocity $APV_{rest}$.

In another example, not illustrated in Fig. 3, the system 100 may be used to calculate the PMM value for the portion of the coronary vessel 120 that is proximal to the reference position 130. In this example, the coronary vessel is a coronary vein, and the PMM value therefore represents the mass of the myocardium 110 from which blood is collected by the portion of the coronary vein. The portion of the coronary vein for which the PMM value is provided in this example is between the reference position 130 and the most proximal extent of the coronary venous vasculature from the reference position 130. In this example the coronary vein may for example be the great cardiac vein, or the middle cardiac vein, or the small cardiac vein, for example. The reference position 130 may in general be any position in a coronary vein.

With reference to Fig. 1 and Fig. 2, in the operation S110, angiographic image data is received. The angiographic image data may be received by the one or more processors 140 illustrated in Fig. 1. The angiographic data may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. In general, the one or more processors may receive the angiographic image data from an angiographic imaging system, or from another source such as a computer readable storage medium, the Internet, or the Cloud, for example.

The angiographic image data includes a temporal sequence of images 150 representing a flow of an injected contrast agent bolus through the coronary vessel 120. In this regard, the contrast agent may include a substance such as Iodine, or a Lanthanide such as Gadolinium, or indeed another substance that provides visibility of a flow in the vasculature into which the contrast agent bolus is injected. The contrast agent may be injected into the vasculature from a syringe. The syringe may be controlled manually, or by a syringe driver. Together, the syringe and the syringe driver are also referred to as an injector. An injection catheter is used to fluidically couple the syringe to the coronary vessel. In-use, a distal end of the injection catheter is inserted into the vasculature in order to inject the contrast agent into the coronary vessel. By way of an example, the contrast agent may be injected into the vasculature using the injector 170 illustrated in Fig. 1. The injector 170 may also be controlled manually, or automatically.

In general, the temporal sequence of images 150 may be generated by an angiographic imaging system such as a projection X-ray imaging system or a volumetric imaging system.

Projection X-ray imaging systems typically include a support arm such as a so-called "C-arm", or an "O-arm", that supports an X-ray source-detector arrangement. Projection X-ray imaging systems may alternatively include a support arm with a different shape to these examples. Projection X-ray imaging systems typically generate projection X-ray images with the support arm held in a static position with respect to an imaging region during the acquisition of image data. In some examples, the temporal sequence of images 150 may be fluoroscopic, i.e. live images. In some examples, temporal sequence of images 150 may be generated using a digital subtraction angiography "DSA" technique, and wherein each image is generated by subtracting from the image the corresponding pixel intensities of a background image. The temporal sequence of images 150 may be generated by the projection X-ray imaging system 180 illustrated in Fig. 1, for example. By way of an example, the temporal sequence of images 150 may be generated by the Philips Azurion 7 X-ray imaging system marketed by Philips Healthcare, Best, The Netherlands.

The temporal sequence of images 150 may alternatively be generated by a volumetric imaging system, such as for example a volumetric X-ray imaging system, or an MRI imaging system. A volumetric X-ray imaging system typically generates image data whilst rotating, or stepping, an X-ray source-detector arrangement around an imaging region, and subsequently reconstructs the image data obtained from multiple rotational angles into a 3D, or volumetric image. Examples of volumetric X-ray imaging systems include computed tomography "CT" imaging systems, cone beam CT "CBCT" imaging systems, and spectral CT imaging systems. In some examples, the temporal sequence of images 150 may be fluoroscopic, i.e. live images. In some examples, the temporal sequence of images 150 may be generated using a digital subtraction angiography "DSA" technique. Alternatively, the temporal sequence of images 150 may be generated by a magnetic resonance imaging "MRI" system. For example, magnetic resonance angiography "MRA" volumetric image data may be generated by injecting a contrast agent into the vasculature and using oscillating magnetic fields at specific resonance frequencies to generate images of various anatomical structures using an MRI imaging system.

With reference to Fig. 1 and Fig. 2, in the operation S120, input data representing a resting systolic blood pressure $sBP_{rest}$ for the coronary vessel 120, is received. In general, the resting systolic blood pressure $sBP_{rest}$ may be measured using various devices. A value for the resting systolic blood pressure $sBP_{rest}$ may be received from one of these devices. Alternatively, a value for the systolic blood pressure may be inputted by a user based on a measurement from one of these devices, or the value may be received from a database.

In one example, the injected contrast agent bolus is injected using an injector 170, and the received input data representing the resting systolic blood pressure $sBP_{rest}$ for the coronary vessel 120, comprises intra-vascular pressure data generated by the injector.

The injector 170 may generate the intra-vascular pressure data using an extracorporeal pressure sensor, or an intra-corporeal pressure sensor. Injectors, such as the injector 170 illustrated in Fig. 1, often include an extra-corporeal pressure sensor that is used to monitor the pressure in a coronary vessel during the injection of the contrast agent. In-use, the extra-corporeal pressure sensor is fluidically coupled to the injection catheter that is disposed in the coronary vessel. Since fluid is incompressible, the extra-corporeal pressure sensor measures the intra-vascular pressure at the distal end of the injection catheter, where the distal end of the injection catheter is exposed to the blood, from an extra-corporeal position. As an alternative to an extra-corporeal sensor, the injection catheter include an intra-vascular pressure sensor that is disposed on a portion of the injection catheter that is inserted into the coronary vessel, and which likewise measures the intra-vascular pressure in the coronary vessel.

In another example, the received input data representing the resting systolic blood pressure $sBP_{rest}$ for the coronary vessel 120, is generated by: an extra-corporeal blood pressure cuff; or an intra-vascular pressure sensing device disposed in the coronary vessel 120. Various extra-corporeal blood pressure cuffs are available for this purpose. The intra-vascular pressure sensing device may for instance be a pressure wire. An example of a pressure wire is the ComboWireXT Guide wire marketed by Philips Healthcare, Best, The Netherlands. The intra-vascular pressure sensing device may be disposed in the ostium of the coronary vessel 120, for example.

In some examples, the received input data representing the resting systolic blood pressure $sBP_{rest}$ for the coronary vessel 120, comprises user input. In these examples, the user may provide the user input via user input device configured to receive user input (not illustrated in Fig. 1), such as a keyboard, a mouse, a touchscreen, and so forth.

In another example, the received input data representing the resting systolic blood pressure $sBP_{rest}$ for the coronary vessel 120, is received from a database. In this example, a value for the resting systolic blood pressure $sBP_{rest}$ may be extracted from the database that represents a previously-measured value for a patient, or an average value for a patient cohort, for example.

Returning to Fig. 1 and Fig. 2, in the operation S130, the one or more processors analyze the angiographic image data in order to determine: an average resting peak blood flow velocity $APV_{rest}$ at the reference position 130, a diameter D of the coronary vessel at the reference position 130, and a resting heart rate $HR_{rest}$ for the coronary vessel 120.

In one example, the average resting peak blood flow velocity $APV_{rest}$ at the reference position 130 may be determined by:

- analyzing the angiographic image data, to identify a proximal position Posa and a distal position $Pos_d$ of a front of the injected contrast agent bolus in the coronary vessel 120;
- estimating, from the angiographic image data, a transit length $d_T$ in the coronary vessel, the transit length representing a distance travelled by the front of the injected contrast agent bolus between the proximal position Posa and the distal position $Pos_d$; and
- calculating the average resting peak blood flow velocity $APV_{rest}$ at the reference position 130, based on the transit length $d_T$ and a transit time $T_T$, the transit time representing the time taken for the injected contrast agent bolus 140 to travel between the identified proximal position Posa in the coronary vessel, and the identified distal position $Pos_d$ in the coronary vessel.

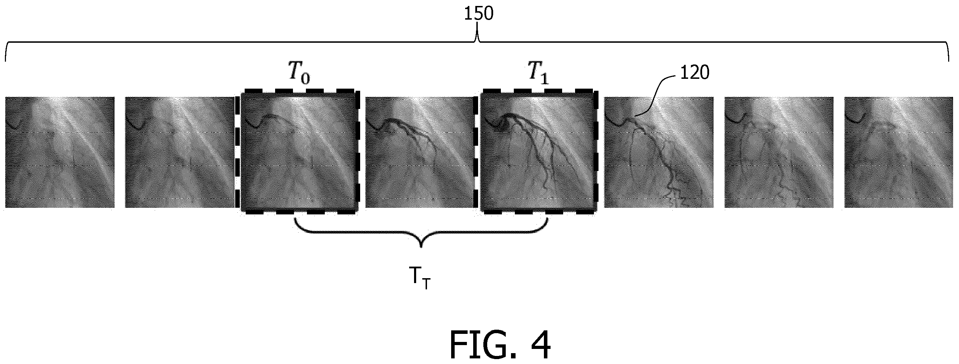

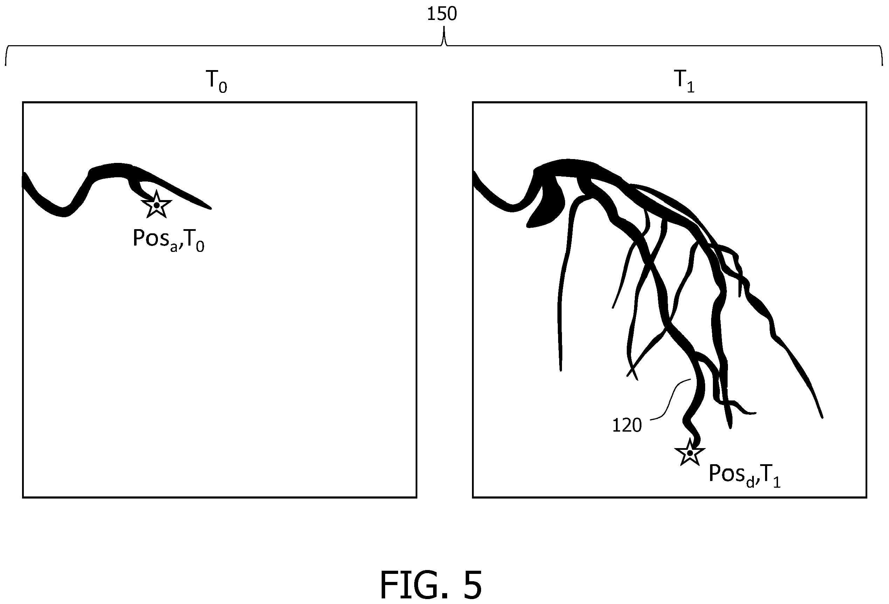

This example is described with reference to Fig. 4, and Fig. 5. Fig. 4 is a schematic diagram illustrating an example of a temporal sequence of images 150 representing a flow of an injected contrast agent bolus through a coronary vessel 120, in accordance with some aspects of the present disclosure. Fig. 5 is a schematic diagram illustrating an earlier image and a later image in a temporal sequence of images 150, in accordance with some aspects of the present disclosure.

With reference to Fig. 4, the temporal sequence of images 150 includes multiple images, including an earlier image at time $T_0$ and a later image at time $T_1$. As time progresses from left to right in the image, the contrast agent can be seen to initially flow into the coronary vessel 120, and then to wash-out of the coronary vessel. In each image, a position of the front of the injected contrast agent bolus may be seen. The coronary vessel indicated in Fig. 120 may for instance be the left coronary artery.

Fig. 5 illustrates the images at times $T_0$ and $T_1$ from Fig. 4 in more detail. The image at time $T_0$ may be referred-to as an earlier image in the temporal sequence of images 150, and the image at time $T_1$ may be referred-to as a later image in the temporal sequence. The positions of the front of the injected contrast agent bolus in the coronary vessel 120 are more clearly visible in the images in Fig. 5 as Posa at time To, and $Pos_d$ at time $T_1$, respectively.

In this example, the angiographic image data, is analyzed in order to identify a proximal position Posa and a distal position $Pos_d$ of a front of the injected contrast agent bolus in the coronary vessel 120. In general, the position of a front of the injected contrast agent may be determined from the angiographic image data by segmenting the images in order to identify the coronary vessel 120, and applying an intensity threshold to the images to identify the position of the front in the images. The proximal position Posa and the distal position $Pos_d$ may be determined by selecting an earlier image at time $T_0$ and a later image at time $T_1$ from the sequence of images 150 such that the positions of the front in the selected images are in the coronary vessel of interest. The positions of the front in the selected images may for example be in the vicinity of the reference position 150, or stenosis. By way of some examples, the earlier image at time $T_0$ may be selected such that the front in this image is at the reference position, or upstream, or downstream of the reference position, and the later image at time $T_1$ may be selected such that the front in this image is downstream of the reference position. In the case that a stenosis is present in the coronary vessel 120, the earlier image may be selected such that the front in this image is at the location of the stenosis, or downstream of the stenosis, and the later image may be selected such that the front in this image is downstream of the stenosis. As mentioned above, the earlier image may be selected such that the position of the front is up to a few millimeters, or up to twenty millimeters downstream of the stenosis in order to avoid the impact of turbulent flow generated by the stenosis on the measured average resting peak blood flow velocity $APV_{rest}$.

In this example, a transit length $d_T$ in the coronary vessel, is estimated from the angiographic image data. The transit length represents a distance travelled by the front of the injected contrast agent bolus between the proximal position Posa and the distal position $Pos_d$. The transit length $d_T$ may be determined by:

- mapping the proximal position Posa from the earlier image to the later image to provide a mapped proximal position Pos'a in the later image, or by mapping the distal position $Pos_d$ from the later image to the earlier image to provide a mapped distal position $Pos'_d$ in the earlier image; and
- determining as the transit length $d_T$, a length of the coronary vessel 120 between the mapped proximal position Pos'a and the distal position $Pos_d$ in the later image, or between the proximal position Posa and the mapped distal position $Pos'_d$ in the earlier image, respectively.

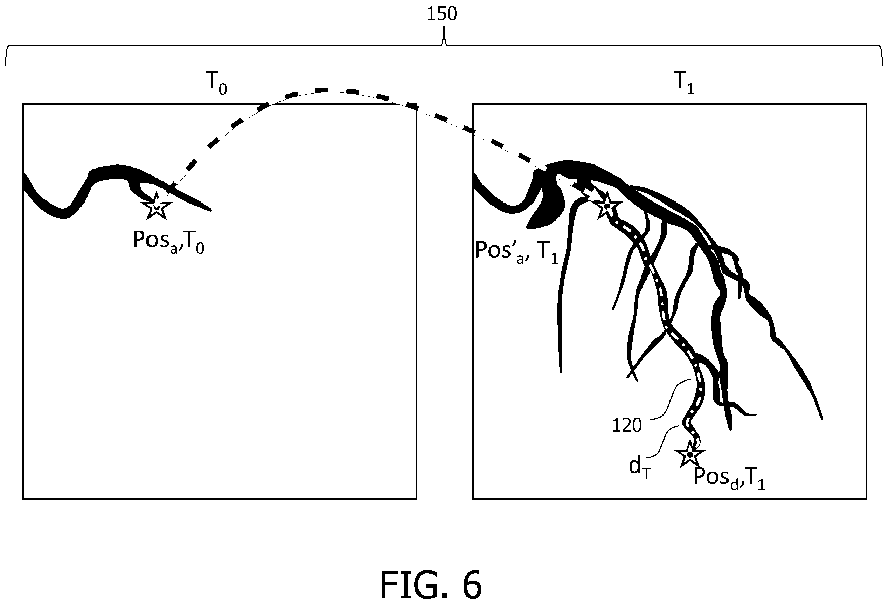

These operations are illustrated with reference to Fig. 6, which is a schematic diagram illustrating the determination of a transit length $d_T$ in a coronary vessel 120 by mapping a proximal position Posa of a front of an injected contrast agent bolus from an earlier image in a temporal sequence of images 150 to a later image in the temporal sequence of images to provide a mapped proximal position Pos'a in the later image, in accordance with some aspects of the present disclosure.

With reference to Fig. 6, the mapping operation may be performed by registering the earlier and later images to one another, and transferring the position from one image to the other. The transit length $d_T$ is indicated in Fig. 6, and its length may be determined based on a known scaling between the inter-pixel spacing in these images and a real-world dimension, such as centimeters. This scaling may be determined by calibrating the imaging system, or it may be calculated based on the geometry of the imaging system with respect to the coronary vessel 120.

In this example, the average resting peak blood flow velocity $APV_{rest}$ at the reference position 130, is then calculated based on the transit length $d_T$ and a transit time $T_T$. This may be achieved by dividing the transit length $d_T$ by the transit time $T_T$. The transit time represents the time taken for the injected contrast agent bolus 140 to travel between the identified proximal position Posa in the coronary vessel, and the identified distal position $Pos_d$ in the coronary vessel. The transit time $T_T$ may be calculated by determining the time difference between the earlier image and the later image, i.e. the difference in time between $T_1$ and $T_0$.

Returning to Fig. 1 and Fig. 2, in the operation S130, the one or more processors also analyze the angiographic image data in order to determine a diameter D of the coronary vessel at the reference position 130.

In one example, the one or more processors are configured to determine the diameter D of the coronary vessel 120 at the reference position 130, by:

- receiving user input specifying a location of the reference position 130 in the angiographic image data; and
- calculating the diameter D of the coronary vessel at the specified location, using the angiographic image data.

In this example, the user input may be received via a user input device, as described above. The system 100 may include a display 190 that displays the earlier image generated at time To, or the later image generated at time $T_1$. The user may for instance specify the reference position in the earlier image at time To, or in the later image at time $T_1$ using the user input device. The diameter D of the coronary vessel may be calculated at the specified location based on a known scaling between the inter-pixel spacing in these images to a real-world dimension, such as centimeters.

In another example, the coronary vessel 120 includes a stenosis. In this example, the reference position 130 is located distal to the stenosis, and the one or more processors are configured to determine the diameter D of the coronary vessel at the reference position 130, by:

- analyzing the angiographic image data to identify a narrowing in the coronary vessel to identify the stenosis;

- specifying as the reference position 130, a location in the coronary vessel distal to the stenosis; and
- calculating the diameter D of the coronary vessel at the specified location, using the angiographic image data.

In this example, the narrowing may be detected using known image processing techniques such as segmentation. Alternatively, a feature detector, or a trained classifier may also be used to detect the narrowing. The operation of specifying the reference position as a location in the coronary vessel distal to the stenosis may also include applying an offset to the identified narrowing. For example, an offset of up to a few millimeters, or up to twenty millimeters may be applied in order that the diameter is measured away from, and distal to, the stenosis. In this example the diameter D of the coronary vessel may be calculated at the specified location based on a known scaling between the inter-pixel spacing in these images and a real-world dimension, such as centimeters.

Returning to Fig. 1 and Fig. 2, in the operation S130, the one or more processors analyze the angiographic image data in order to determine a resting heart rate $HR_{rest}$ for the coronary vessel 120.

The one or more processors may determine the resting heart rate $HR_{rest}$ for the coronary vessel 120, by:

- detecting a pulsation of a feature in the angiographic image data;
- extracting a heart rate signal representing the pulsation; and
- determining the resting heart rate $HR_{rest}$ based on a period of the extracted heart rate signal.

The feature in the angiographic image data may for instance be a coronary artery, a portion of the myocardium, a cardiac shadow, or the distal end of an injection catheter. These features may be identified in the angiographic image data manually by a user, or automatically using image processing techniques. A neural network, or a feature detector may be provided in order to detect these features. These features typically pulsate as a result of cardiac-induced motion. A heart rate signal may be extracted by analysing the pixel intensities corresponding to these features by for example summing their values together, or averaging them. Subsequently signal processing techniques may be used to determine a period of the extracted heart rate signal, and thus determine the resting heart rate $HR_{rest}$.

By way of another example, a dimension of the coronary arteries may be detected in the temporal sequence of images and used to provide the heart rate signal. In this respect, techniques disclosed in a document by Ciusdel C., et al., entitled "Deep neural networks for ECG-free cardiac phase and end-diastolic frame detection on coronary angiographies", Comput Med Imaging Graph. 2020 Sep;84:101749, may be used to determine the resting heart rate $HR_{rest}$.

Returning to Fig. 1 and Fig. 2, in the operation S140, the one or more processors calculate the PMM value for the coronary vessel 120, based on: the determined average resting peak blood flow velocity $APV_{rest}$, the determined diameter D, the determined resting heart rate $HR_{rest}$, and the resting systolic blood pressure $sBP_{rest}$.

In one example, the PMM value is calculated using Equation 1.

In another example, the PMM value is calculated using Equation 2 below:

$$PMM = \frac{APV_{rest} \times D^2 \times \pi}{1.24 \times 10^{-3} \times HR_{rest} \times sBP_{rest} + 1.6} \times \emptyset$$

The parameters in Equation 2 represent the same quantities as described above for Equation 1. As compared to Equation 1, Equation 2 additionally includes the parameter $\phi$, which represents a correction factor. Using the correction factor $\phi$ in this Equation may be considered to provide a more accurate PMM value that compensates for factors such as the average resting peak blood flow velocity $APV_{rest}$ being measured from angiographic images as opposed to using an invasive technique.

In this example, the correction factor $\phi$ may be determined by:

- inputting into a neural network 160: the average resting peak blood flow velocity $APV_{rest}$ at the reference position 130, the diameter D of the coronary vessel at the reference position 130, the resting heart rate $HR_{rest}$ for the coronary vessel, and the resting systolic blood pressure $sBP_{rest}$ for the coronary vessel; and
- generating the correction factor $\phi$ in response to the inputting; and
- wherein the neural network 160 is trained to generate the correction factor $\phi$, based on the inputted average resting peak blood flow velocity $APV_{rest}$, the diameter D, the resting heart rate $HR_{rest}$, and the resting systolic blood pressure $sBP_{rest}$.

Generating the correction factor $\phi$ using a neural network may be considered to provide a more accurate correction factor since it is based on multiple parameters, i.e. the average resting peak blood flow velocity $APV_{rest}$, the diameter D, the resting heart rate $HR_{rest}$, and the resting systolic blood pressure $sBP_{rest}$. Thus, it may be considered to provide a PMM value that accurately compensates for the average resting peak blood flow velocity $APV_{rest}$ being measured from angiographic images as opposed to using an invasive technique.

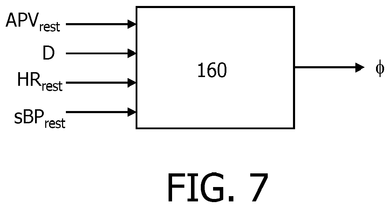

Fig. 7 is a schematic diagram illustrating an example of a trained neural network 160 for calculating a correction factor $\phi$ for correcting a PMM value, in accordance with some aspects of the present disclosure.

The neural network 160 may be trained to generate the correction factor $\phi$, by:

- receiving training data for a plurality of patients, the training data for each patient comprising: an average resting peak blood flow velocity $APV_{rest}$ at the reference position 130 determined from angiographic image data, a diameter D of the coronary vessel 120 at the reference position 130 determined from angiographic image data, a resting heart rate $HR_{rest}$ for the coronary vessel determined from angiographic image data, and a resting systolic blood pressure $sBP_{rest}$ for the coronary vessel;
- receiving ground truth data corresponding to the training data, the ground truth data comprising: an average resting peak blood flow velocity $APV_{rest}$ at the reference position 130 determined from an intra-vascular flow sensor, a diameter D of the coronary vessel at the reference position 130 determined from angiographic image data, a resting heart rate $HR_{rest}$ for the coronary vessel determined from a heart rate sensor, and a resting systolic blood pressure $sBP_{rest}$ for the coronary vessel; and
- for each of a plurality of the patients:
  inputting the training data into the neural network 160; and adjusting parameters of the neural network 160 until a difference between i) a product of a correction factor generated by the neural network 160, and a PMM value calculated using the training data, and ii) a PMM value calculated using the corresponding ground truth data, meets a stopping criterion.

The plurality of patients represented in the training data may be selected from a range of ages, and thus the training data may represent patients diagnosed as having CAD, NOCAD, and patients without any form of CAD, different BMI, and so forth.

In general, the training of a neural network involves inputting a training dataset into the neural network, and iteratively adjusting the neural network's parameters until the trained neural network provides an accurate output. Training is often performed using a Graphics Processing Unit "GPU" or a dedicated neural processor such as a Neural Processing Unit "NPU" or a Tensor Processing Unit "TPU". Training often employs a centralized approach wherein cloud-based or mainframe-based neural processors are used to train a neural network. Following its training with the training dataset, the trained neural network may be deployed to a device for analyzing new input data during inference. The processing requirements during inference are significantly less than those required during training, allowing the neural network to be deployed to a variety of systems such as laptop computers, tablets, mobile phones and so forth. Inference may for example be performed by a Central Processing Unit "CPU", a GPU, an NPU, a TPU, on a server, or in the cloud.

The process of training the neural network 160 described above therefore includes adjusting its parameters. The parameters, or more particularly the weights and biases, control the operation of activation functions in the neural network. In supervised learning, the training process automatically adjusts the weights and the biases, such that when presented with the input data, the neural network accurately provides the corresponding expected output data. In order to do this, the value of the loss functions, or errors, are computed based on a difference between predicted output data and the expected output data. The value of the loss function may be computed using functions such as the negative log-likelihood loss, the mean squared error, the Huber loss, or the cross entropy loss. During training, the value of the loss function is typically minimized, and training is terminated when the value of the loss function satisfies a stopping criterion. Sometimes, training is terminated when the value of the loss function satisfies one or more of multiple criteria.

Various methods are known for solving the loss minimization problem such as gradient descent, Quasi-Newton methods, and so forth. Various algorithms have been developed to implement these methods and their variants including but not limited to Stochastic Gradient Descent "SGD", batch gradient descent, mini-batch gradient descent, Gauss-Newton, Levenberg Marquardt, Momentum, Adam, Nadam, Adagrad, Adadelta, RMSProp, and Adamax "optimizers" These algorithms compute the derivative of the loss function with respect to the model parameters using the chain rule. This process is called backpropagation since derivatives are computed starting at the last layer or output layer, moving toward the first layer or input layer. These derivatives inform the algorithm how the model parameters must be adjusted in order to minimize the error function. That is, adjustments to model parameters are made starting from the output layer and working backwards in the network until the input layer is reached. In a first training iteration, the initial weights and biases are often randomized. The neural network then predicts the output data, which is likewise, random. Backpropagation is then used to adjust the weights and the biases. The training process is performed iteratively by making adjustments to the weights and biases in each iteration. Training is terminated when the error, or difference between the predicted output data and the expected output data, is within an acceptable range for the training data, or for some validation data. Subsequently the neural network may be deployed, and the trained neural network makes predictions on new input data using the trained values of its parameters. If the training process was successful, the trained neural network accurately predicts the expected output data from the new input data.

It is noted that in some examples, the system 100 described above may also include one or more of: an X-ray imaging system 180 for providing the angiographic image data, an injector 170 for injecting a contrast agent, a display 190 for displaying the calculated PMM value, the angiographic image data, and so forth, a patient bed 200, and a user input device configured to receive user input (not illustrated in Fig. 1), such as a keyboard, a mouse, a touchscreen, and so forth.

In another example, a computer-implemented method of calculating a partial myocardial mass, PMM, value, is provided. The PMM value represents a mass of a myocardium 110 associated with a portion of a coronary vessel 120, the portion of the coronary vessel being defined relative to a reference position 130 in the coronary vessel. The method comprises:

- receiving S110 angiographic image data comprising a temporal sequence of images 150 representing a flow of an injected contrast agent bolus through the coronary vessel 120;
- receiving S120 input data representing a resting systolic blood pressure $sBP_{rest}$ for the coronary vessel 120;
- analysing S130 the angiographic image data to determine: an average resting peak blood flow velocity $APV_{rest}$ at the reference position 130, a diameter D of the coronary vessel at the reference position 130, and a resting heart rate $HR_{rest}$ for the coronary vessel 120; and
- calculating S140 the PMM value for the portion of the coronary vessel 120, based on: the determined average resting peak blood flow velocity $APV_{rest}$, the determined diameter D, the determined resting heart rate $HR_{rest}$, and the resting systolic blood pressure $SBP_{rest}$.

In another example, a computer program product, is provided. The computer program product comprises instructions which when executed by one or more processors, cause the one or more processors to carry out a method of calculating a partial myocardial mass, PMM, value. The PMM value represents a mass of a myocardium 110 associated with a portion of a coronary vessel 120, the portion of the coronary vessel being defined relative to a reference position 130 in the coronary vessel. The method comprises:

- receiving S110 angiographic image data comprising a temporal sequence of images 150 representing a flow of an injected contrast agent bolus through the coronary vessel 120;
- receiving S120 input data representing a resting systolic blood pressure $sBP_{rest}$ for the coronary vessel 120;
- analysing S130 the angiographic image data to determine: an average resting peak blood flow velocity $APV_{rest}$ at the reference position 130, a diameter D of the coronary vessel at the reference position 130, and a resting heart rate $HR_{rest}$ for the coronary vessel 120; and
- calculating S140 the PMM value for the portion of the coronary vessel 120, based on: the determined average resting peak blood flow velocity $APV_{rest}$, the determined diameter D, the determined resting heart rate $HR_{rest}$, and the resting systolic blood pressure $sBP_{rest}$.

The above examples are to be understood as illustrative of the present disclosure, and not restrictive. Further examples are also contemplated. For instance, the examples described in relation to the system 100, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

**Claims**

1. A system (100) for calculating a partial mass of an organ (110) associated with a portion of a vessel (120), the portion of the vessel being defined relative to a reference position (130) in the vessel, the system comprising one or more processors (140) configured to:

    receive (S110) image data comprising a temporal sequence of images (150) representing a flow of an injected contrast agent bolus through the vessel (120);

receive (S120) input data representing a resting systolic blood pressure ($sBP_{rest}$) for the vessel (120);

analyze (S130) the image data to determine: an average resting peak blood flow velocity ($APV_{rest}$) at the reference position (130), a diameter (D) of the vessel at the reference position (130), and a resting heart rate ($HR_{rest}$) for the vessel (120); and

calculate (S140) the partial mass of the organ (110) associated with the portion of the vessel (120) based on: the determined average resting peak blood flow velocity ($APV_{rest}$), the determined diameter (D), the determined resting heart rate ($HR_{rest}$), and the resting systolic blood pressure ($sBP_{rest}$).

2. The system according to claim 1, wherein the one or more processors are configured to determine the diameter (D) of the vessel (120) at the reference position (130), by:

receiving user input specifying a location of the reference position (130) in the image data; and

calculating the diameter (D) of the vessel at the specified location, using the image data.

3. The system according to claim 1 or 2, wherein the vessel (120) comprises a stenosis, and wherein the reference position (130) is located distal to the stenosis, and wherein the one or more processors are configured to determine the diameter (D) of the vessel at the reference position (130), by:

analysing the image data to identify a narrowing in the vessel to identify the stenosis;

specifying as the reference position (130), a location in the vessel distal to the stenosis; and

calculating the diameter (D) of the vessel at the specified location, using the image data.

4. . The system according to any one of claims 1 to 3, wherein the one or more processors are configured to determine the resting heart rate ($HR_{rest}$) for the vessel (120), by:

detecting a pulsation of a feature in the image data;

extracting a heart rate signal representing the pulsation; and

determining the resting heart rate ($HR_{rest}$) based on a period of the extracted heart rate signal.

5. The system according to any one of claims 1 to 4, wherein the injected contrast agent bolus is injected using an injector (170); and wherein the received input data representing the resting systolic blood pressure ($sBP_{rest}$) for the vessel (120), comprises intra-vascular pressure data generated by the injector.

6. The system according to any one of claims 1 to 4, wherein the received input data representing a resting systolic blood pressure ($sBP_{rest}$) for the vessel (120) comprises user input, or is received from a database, or is generated by:

an extra-corporeal blood pressure cuff; or

an intra-vascular pressure sensing device disposed in the vessel (120).

7. The system according to any previous claim, wherein the one or more processors are configured to determine the average resting peak blood flow velocity ($APV_{rest}$) at the reference position (130), by:

analysing the angiographic image data, to identify a proximal position (Posa) and a distal position (Posd) of a front of the injected contrast agent bolus in the vessel (120);

estimating, from the image data, a transit length ($d_T$) in the vessel, the transit length representing a distance travelled by the front of the injected contrast agent bolus between the proximal position (Posa) and the distal position (Posd); and

calculating the average resting peak blood flow velocity ($APV_{rest}$) at the reference position (130), based on the transit length ($d_T$) and a transit time ($T_T$), the transit time representing the time taken for the injected contrast agent bolus (140) to travel between the identified proximal position (Posa) in the vessel, and the identified distal position ($Pos_d$) in the vessel.

8. The system according to claim 7, wherein the proximal position (Posa) corresponds to a position of the front of the injected contrast agent bolus (140) in the vessel (120) in an earlier image in the temporal sequence (130), and wherein the distal position ($Pos_d$) corresponds to a position of the detected front in a later image in the temporal sequence (130); and wherein the one or more processors are configured to:

determine the transit time ($T_T$) based on the time difference between the later image and the earlier image; and

determine the transit length ($d_T$) by:

mapping the proximal position (Posa) from the earlier image to the later image to provide a mapped proximal position (Pos'a) in the later image, or by mapping the distal position (Posd) from the later image to the earlier image to provide a mapped distal position (Pos'd) in the earlier image; and
determining as the transit length ($d_T$), a length of the vessel (120) between the mapped proximal position (Pos'a) and the distal position (Posd) in the later image, or between the proximal position (Posa) and the mapped distal position (Pos'd) in the earlier image, respectively.

9. The system according to any of the preceding claims, wherein the one or more processors are configured to calculate the partial mass for the portion of the vessel (120) based on a correction factor ($\phi$), and wherein the correction factor is determined by:

inputting into a neural network (160): the average resting peak blood flow velocity ($APV_{rest}$) at the reference position (130), the diameter (D) of the vessel at the reference position (130), the resting heart rate ($HR_{rest}$) for the vessel, and the resting systolic blood pressure ($sBP_{rest}$) for the vessel; and
generating the correction factor ($\phi$) in response to the inputting; and
wherein the neural network (160) is trained to generate the correction factor ($\phi$), based on the inputted average resting peak blood flow velocity ($APV_{rest}$), the diameter (D), the resting heart rate ($HR_{rest}$), and the resting systolic blood pressure ($sBP_{rest}$).

10. The system according to claim 9, wherein the neural network (160) is trained to generate the correction factor ($\phi$), by:

receiving training data for a plurality of patients, the training data for each patient comprising: an average resting peak blood flow velocity ($APV_{rest}$) at the reference position (130) determined from image data, a diameter (D) of the vessel (120) at the reference position (130) determined from image data, a resting heart rate ($HR_{rest}$) for the vessel determined from image data, and a resting systolic blood pressure ($sBP_{rest}$) for the vessel;
receiving ground truth data corresponding to the training data, the ground truth data comprising: an average resting peak blood flow velocity ($APV_{rest}$) at the reference position (130) determined from an intra-vascular flow sensor, a diameter (D) of the vessel at the reference position (130) determined from image data, a resting heart rate ($HR_{rest}$) for the vessel determined from a heart rate sensor, and a resting systolic blood pressure ($sBP_{rest}$) for the vessel; and
for each of a plurality of the patients:
inputting the training data into the neural network (160); and adjusting parameters of the neural network (160) until a difference between i) a product of a correction factor generated by the neural network (160), and a partial mass calculated using the training data, and ii) a partial mass calculated using the corresponding ground truth data, meets a stopping criterion.

11. The system of any of the preceding claims, wherein the image data is angiographic image data provided by an X-ray imaging system or a magnetic resonance imaging system.

12. The system of any of the preceding claims, wherein the organ is a heart and the partial mass of the organ is a partial myocardial mass, PMM, value.

13. The system according to claim 12, wherein:
the portion of the vessel (120) is distal to the reference position (130), wherein the vessel is a coronary artery, and wherein the PMM value represents the mass of the myocardium (110) supplied by the portion of the coronary artery

14. The system according to claims 12, wherein:
the portion of the vessel (120) is proximal to the reference position (130), wherein the vessel is a coronary vein, and wherein the PMM value represents the mass of the myocardium (110) from which blood is collected by the portion of the coronary vein.

15. A method of calculating a partial mass of an organ (110) associated with a portion of a vessel (120), the portion of the vessel being defined relative to a reference position (130) in the vessel, the method comprising:

receiving (S110) image data comprising a temporal sequence of images (150) representing a flow of an injected contrast agent bolus through the vessel (120);

receiving (S120) input data representing a resting systolic blood pressure ($sBP_{rest}$) for the vessel (120);

analyzing (S130) the image data to determine: an average resting peak blood flow velocity ($APV_{rest}$) at the reference position (130), a diameter (D) of the vessel at the reference position (130), and a resting heart rate ($HR_{rest}$) for the vessel (120); and

calculating (S140) the partial mass of the organ (110) associated with the portion of the vessel (120) based on: the determined average resting peak blood flow velocity ($APV_{rest}$), the determined diameter (D), the determined resting heart rate ($HR_{rest}$), and the resting systolic blood pressure ($sBP_{rest}$).

100

180

190

170

200

140

# FIG. 1

S110

S120

S130

S140

# FIG. 2

FIG. 3

FIG. 4

150

T_0

Pos_a,T_0

T_1

120

Pos_d,T_1

FIG. 5

FIG. 6

EP 4 197 429 A1

$APV_{rest}$ $\longrightarrow$

$D$ $\longrightarrow$

$HR_{rest}$ $\longrightarrow$

$sBP_{rest}$ $\longrightarrow$

160

$\longrightarrow \phi$

FIG. 7

**EUROPEAN SEARCH REPORT**

Europäisches Patentamt
European Patent Office
Office européen des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | MURAI TADASHI ET AL: "Quantification of Myocardial Mass Subtended by a Coronary Stenosis Using Intracoronary Physiology", [CIRCULATION / CARDIOVASCULAR INTERVENTIONS] CIRCULATION : JOURNAL OF THE AMERICAN HEART ASSOCIATION, vol. 12, no. 8, 1 August 2019 (2019-08-01), XP055914239, US ISSN: 1941-7640, DOI: 10.1161/CIRCINTERVENTIONS.118.007322 | 1-3,5,6, 9-15 | INV. A61B5/00 A61B5/021 A61B5/024 A61B5/026 A61B5/107 A61B6/00 |
| Y | * pages 1-4 * <br> * figures 2, 3 * | 4,7,8 | |
| Y | US 2017/354383 A1 (ODESSKY ADAM [US] ET AL) 14 December 2017 (2017-12-14) <br> * paragraph [0048] * | 4 | |
| A | US 2002/161302 A1 (PATERNOSTRO GIOVANNI [US]) 31 October 2002 (2002-10-31) <br> * paragraph [0048] * | 4 | |
| | | | **TECHNICAL FIELDS SEARCHED (IPC)** |
| Y | US 2021/236000 A1 (HUO YUNFEI [CN] ET AL) 5 August 2021 (2021-08-05) <br> * paragraphs [0029] - [0033] * | 7,8 | A61B |
| A | US 2019/357778 A1 (WILSON ROBERT F [US] ET AL) 28 November 2019 (2019-11-28) <br> * paragraph [0005] * | 7,8 | |
| A | US 2020/337773 A1 (RAWLINSON ANDREW [AU] ET AL) 29 October 2020 (2020-10-29) <br> * paragraphs [0040], [0041] * | 7,8 | |

-/--

The present search report has been drawn up for all claims

| Place of search | Date of completion of the search | Examiner |
|---|---|---|
| Munich | 3 May 2022 | Trattner, Barbara |

EPO FORM 1503 03.82 (P04C01)

page 1 of 2

Europäisches
Patentamt

European
Patent Office

Office européen
des brevets

# EUROPEAN SEARCH REPORT

Application Number

**EP 21 21 4244**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KURATA AKIRA ET AL: "Quantification of the myocardial area at risk using coronary CT angiography and Voronoi algorithm-based myocardial segmentation", EUROPEAN RADIOLOGY, SPRINGER BERLIN HEIDELBERG, BERLIN/HEIDELBERG, vol. 25, no. 1, 31 August 2014 (2014-08-31), pages 49-57, XP035382190, ISSN: 0938-7994, DOI: 10.1007/S00330-014-3388-2 [retrieved on 2014-08-31] * the whole document * | 1 | |
| A | IDE SEIKO ET AL: "Cardiac computed tomography-derived myocardial mass at risk using the Voronoi-based segmentation algorithm: A histological validation study", JOURNAL OF CARDIOVASCULAR COMPUTED TOMOGRAPHY, vol. 11, no. 3, 1 May 2017 (2017-05-01), pages 179-182, XP055914406, AMSTERDAM, NL ISSN: 1934-5925, DOI: 10.1016/j.jcct.2017.04.007 * the whole document * | 1 | TECHNICAL FIELDS SEARCHED (IPC) |

The present search report has been drawn up for all claims

| Place of search | Date of completion of the search | Examiner |
|---|---|---|
| Munich | 3 May 2022 | Trattner, Barbara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
................................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 21 21 4244

This annex lists the patent family members relating to the patent documents cited in the above-mentioned European search report.
The members are as contained in the European Patent Office EDP file on
The European Patent Office is in no way liable for these particulars which are merely given for the purpose of information.

03-05-2022

| Patent document cited in search report | | Publication date | Patent family member(s) | | Publication date |
|---|---|---|---|---|---|
| US 2017354383 | A1 | 14-12-2017 | NONE | | |
| US 2002161302 | A1 | 31-10-2002 | NONE | | |
| US 2021236000 | A1 | 05-08-2021 | CN | 112384136 A | 19-02-2021 |
| | | | CN | 112384137 A | 19-02-2021 |
| | | | EP | 3881758 A1 | 22-09-2021 |
| | | | JP | 2022506325 A | 17-01-2022 |
| | | | US | 2021236000 A1 | 05-08-2021 |
| | | | WO | 2020098704 A1 | 22-05-2020 |
| | | | WO | 2020098705 A1 | 22-05-2020 |
| US 2019357778 | A1 | 28-11-2019 | CN | 112204609 A | 08-01-2021 |
| | | | EP | 3797399 A1 | 31-03-2021 |
| | | | JP | 2021525120 A | 24-09-2021 |
| | | | US | 2019357778 A1 | 28-11-2019 |
| | | | WO | 2019226849 A1 | 28-11-2019 |
| US 2020337773 | A1 | 29-10-2020 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MURAI, T. et al.** Quantification of Myocardial Mass Subtended by a Coronary Stenosis Using Intracoronary Physiology. *Circ Cardiovasc Interv.,* 2019, vol. 12, e007322

- **CIUSDEL C. et al.** Deep neural networks for ECG-free cardiac phase and end-diastolic frame detection on coronary angiographies. *Comput Med Imaging Graph.,* September 2020, vol. 84, 101749